# EUROPEAN PATENT SPECIFICATION

(11) **EP 3 874 067 B1**
(45) Date of publication and mention of the grant of the patent: **03.04.2024**
(21) Application number: 19878745.9
(22) Date of filing: 30.10.2019
(51) Int. Cl.: C12Q 1/6825, C12N 15/115, B82Y 30/00

(54) **SELF-ASSEMBLING NUCLEIC ACID SURFACES FOR BIOSENSOR APPLICATIONS**
SELBSTASSEMBLIERENDE NUKLEINSÄUREOBERFLÄCHEN FÜR BIOSENSORANWENDUNGEN
SURFACES D'ACIDE NUCLÉIQUE À AUTO-ASSEMBLAGE POUR APPLICATIONS DE BIOCAPTEURS

(30) Priority: 30.10.2018 US 201862752469 P
(43) Date of publication of application: 08.09.2021
(73) Proprietor: Institut National De La Recherche Scientifique, Québec, Québec G1K 9A9 (CA)
(72) Inventor: PERREAULT, Jonathan, Ste-Geneviève, Québec H9H 1E3 (CA); WALSH, Ryan, Montreal, Québec H2W 2M7 (CA)
(74) Representative: Jacob, Reuben Ellis
(86) International application number: PCT/CA2019/051534
(87) International publication number: WO 2020/087166

(56) References cited:
- WO-A1-2014/018675
- US-A1- 2003 087 262
- US-A1- 2006 286 553
- ZHANG et al.: "Self-Assembly-Based Structural DNA Nanotechnology", Current Organic Chemistry, vol. 15, no. 4, 2011, pages 534-547, XP055706496, ISSN: 1875-5348

## Description

### BACKGROUND

### (a) Field

The subject matter disclosed generally relates to nucleic acid structures, surfaces and sensors comprising the structures, and processes for making the same. More particularly, the invention relates to nucleic acid structures comprising annealed motifs made from complementary oligonucleotides, which may be anchored to surfaces, and sensors made therefrom. The present invention also describes a methods to generate the nucleic acid structures.

### (b) Related Prior Art

The current biosensor production method rely on the sequential assembly of detection elements, it is clear that uniform evenly distributed attachment sites with proper orientation will maximize recognition of element-target interactions and will increase efficiency, sensitivity and reliability of such sensors.

This invention implies the synthesis of different but pairing-driven complementary oligonucleotides that sequential addition will self-assembled as a nanoconstruction of highly predictable structures to optimize molecular recognition.

For example, immunodiagnostics, protein biochips, and biosensors employed for antigen detection and quantification from biological samples often employ recognition proteins such as antibodies. Ideally, the immobilized antibody is oriented such that the active site, sometimes referred to as the head, has access to the substrate, over the non-active site area of antibody, sometimes referred to as tail. However, randomly immobilized antibodies may assume various surface orientations, including those loosely referred to as side. Improper orientation blocks access to the antibody recognition sites and leads to lower detection efficiency, loss of sensitivity and a propensity for generating false negative as well as false positive results.

This phenomenon of inefficient assembly of detection molecules happens with most of the current types of biological material which are used in biosensor production, including for example aptamers, receptors, and others. Building a three-dimensional structure that binds receptors in a specific and predetermined orientation and spacing introduces a new level of control for these constructs, and opens the possibility of properly orienting the detection molecules for their functional purpose.

In this application, the inventors have identified a combination of self-assembled DNA, RNA or peptide nucleic acids (PNA) layers that allow the production of binding sites that are evenly and regularly spaced. This allows the production of a well-structured surface in a controlled and predictable manner allowing the attachment of different receptors or molecules for sensing devices. Such construction also allows fine control of the binding detection material, permitting receptor concentration estimates enabling biosensor optimization and calibration.

Such biosensor can then be read and analyzed according to different possible technologies, such as but not limited to electrochemical detection, surface plasmon resonance (SPR), dual polarization interferometry, X-ray photoelectron spectroscopy, neutron reflectometry, spectroscopic eMipsometry, atomic force microscopy, time-of-flight secondary-ion mass spectrometry, quartz crystal microbalance and diptest techniques. Enzymatic and fluorescence detection methods are also compatible with this approach.

Zhang et al.: "Self-Assembly-Based Structural DNA Nanotechnology",Current Organic Chemistry, 2011, 15(4):534-547 discloses a DNA strand structure based on double cross-over between 4 separate strands.

### SUMMARY

According to an embodiment, there is provided a nucleic acid structure comprising a plurality of annealed motifs, each of the annealed motifs comprising at least three oligonucleotides, each of the at least three oligonucleotides comprising a respective first, second and third domain, each of the first domain of a respective oligonucleotide of the at least three oligonucleotides being complementary for base pairing with a single one of the second domain of another oligonucleotide of the at least three oligonucleotides, to form the annealed motifs; and each of the third domain of a respective oligonucleotide of the at least three oligonucleotides being complementary for base pairing with the third domain of another oligonucleotide from a different annealed motif, to form nucleic acid structure.

The at least three oligonucleotides may comprise at least three domains. The at least three oligonucleotides may have equal length. The any one of the first, second and third domain of any one of the at least three oligonucleotides may have a different length.

The any one of the at least three oligonucleotides may be 18-180 nucleotides in length.

The nucleic acid structure may further comprise a first anchoring moiety, for anchoring of the nucleic acid structure to a solid support.

The nucleic acid structure may further comprise a second anchoring moiety, for anchoring a functional element to the nucleic acid structure.

The first or second anchoring moiety may be a thiol group, an amide group, a diazonium group, an azido group, an alkyne group, a nanotube, a quantum dot, a metal, a silicon, an oligonucleotide, a peptide, a biotin, and combinations thereof.

The oligonucleotide may be a polyT(25), a polyA(25), and combinations thereof.

The first or the second anchoring moiety may be attached to an oligonucleotide having a nucleotide sequence complementary for the first domain or the second domain of another oligonucleotide of the annealed motif.

The first or the anchoring moiety further comprises a functional element.

The functional element may comprise a nucleic acid moiety, a protein moiety, a peptide moiety, a polysaccharide moiety, a microorganism moiety, a nanoparticle moiety, or combinations thereof.

The nucleic acid moiety may be an aptamer domain.

The nucleic acid moiety may comprise DNA, RNA or PNA.

The any one of the first, second and third domain, or a combination thereof, of any one of the at least three oligonucleotides may further comprise an aptamer domain.

The aptamer domain may be configured to bind to an antigen.

The nucleic acid moiety may be a nucleic acid structure according to the present invention.

The protein moiety may be an antibody, an antigen binding domain thereof, or a fusion protein thereof.

The annealed motif from the plurality of annealed motifs forms a functional element.

The annealed motif from the plurality of annealed motifs further comprises a functional element incorporated in any oligonucleotide of the annealed motif.

The functional element may be an aptamer domain.

The solid support may be a metallic surface, a silicon surface, a polymer surface, and combinations thereof.

The metallic surface may be a gold surface, a platinum surface, an iron surface, a steel surface, a copper surface, or combinations thereof.

The silicon surface may be a quartz surface, a glass surface, a polymerized siloxane surface, or combinations thereof.

The polymer surface may be a cellulose surface, a starch surface, a nitrocellulose surface, a chitin surface, a plastic surface.

The cellulose may be a carboxymethyl cellulose surface.

According to another embodiment, there is provided a surface comprising a solid support and a nucleic acid structure according to the present invention attached thereon.

The surface may be a metallic surface, a silicon surface, a polymer surface, and combinations thereof.

The metallic surface may be a gold surface, a platinum surface, an iron surface, a steel surface, a copper surface, or combinations thereof.

The silicon surface may be a quartz surface, a glass surface, a polymerized siloxane surface, or combinations thereof.

The polymer surface may be a cellulose surface, a starch surface, a nitrocellulose surface, a chitin surface, a plastic surface.

The cellulose may be a carboxymethyl cellulose surface.

According to another embodiment, there is provided a method for producing a nucleic acid structure from at least first and second annealed motifs, each annealed motif comprising at least a first, second and third oligonucleotide each comprising a respective first, second and third domain,
each of the first domain of a respective oligonucleotide of the at least a first, second and third oligonucleotide being complementary for base pairing with a single one of the second domain of another of the at least first, second or third oligonucleotide, to form the annealed motif; and
at least one of the third domain of a respective oligonucleotide of the at least first, second or third oligonucleotide of the first annealed motif being complementary for base pairing with at least one third domain of one of the at least first, second or third oligonucleotide from the second annealed motif, to form nucleic acid structure,
the method comprising step a) :
mixing in alternation an amount of the first annealed motif with the second annealed motif for a time sufficient to form the nucleic acid structure.

The one of the first or the second annealed motif may be an anchored annealed motif, anchored to a solid support.

The anchored annealed motif may be anchored to the solid support by step a') before step a):
a') deposition on the solid support of an oligonucleotide C' having a first domain and a first anchoring moiety, for anchoring of the oligonucleotide C' to a solid support configured to react with the first anchoring moiety,
followed by mixing of an oligonucleotide A having a second domain complementary for base pairing with the first domain of oligonucleotide C',
followed by mixing of an oligonucleotide B having a second domain complementary for base pairing with a first domain of oligonucleotide A, and
followed by mixing of an oligonucleotide C" having a second domain complementary for base pairing with a first domain of oligonucleotide B,
to form the anchored annealed motif;
wherein each of the oligonucleotide A, B and C" have a respective third domain complementary for base pairing with at least one third domain of another oligonucleotide from a different annealed motif.

The annealed motif from the at least first and second annealed motifs forms a functional element.

The annealed motif from the at least first and second annealed motifs may further comprises a functional element incorporated in any oligonucleotide of the annealed motif.

The one of the at least first or the second annealed motif may be an anchorage annealed motif further comprises a second anchoring moiety, for anchoring a functional element to the nucleic acid structure.

The functional element may be an aptamer domain.

The functional element comprises a nucleic acid moiety, a protein moiety, a peptide moiety, a polysaccharide moiety, a microorganism moiety, or combinations thereof.

The anchorage annealed motif may be prepared by
a") mixing an oligonucleotide D' having a first domain and a second anchoring moiety, for anchoring a functional element to the oligonucleotide D',
followed by mixing of an oligonucleotide E having a second domain complementary for base pairing with the first domain of oligonucleotide D',
followed by mixing of an oligonucleotide F having a second domain complementary for base pairing with a first domain of oligonucleotide E, and
followed by mixing of an oligonucleotide D" having a second domain complementary for base pairing with a first domain of oligonucleotide F,
to form the anchorage annealed motif;
wherein each of the oligonucleotide D", E and F have a respective third domain complementary for base pairing with at least one third domain of another oligonucleotide from a different annealed motif.

The oligonucleotide C' or the oligonucleotide D' further comprises a functional element.

The functional element comprises a nucleic acid moiety, a protein moiety, a peptide moiety, a polysaccharide moiety, a microorganism moiety, a nanoparticle moiety, or combinations thereof.

According to another embodiment, there is provided a sensor for the detection of an analyte comprising the nucleic acid motif of the present invention, or the surface of any one of claims of the present invention, in communication with a system for detecting a physical change when the analyte interacts with the nucleic acid motif or the surface.

The physical change is a change in surface plasmon resonance, a surface plasmon resonance wavelength shift, a change in electrical signal, a change in electrochemical signal, a change in a color signal, a change in fluorescence signal, a change in a luminescence signal, an acoustic change, and a change in density, and combinations thereof..

According to another embodiment, there may be provided a method of detecting an analyte comprising detecting a physical change with a sensor comprising the nucleic acid motif of the present invention, or the surface of the present invention, in communication with a system for detecting the physical change.

The physical change is a change in surface plasmon resonance, a surface plasmon resonance wavelength shift, a change in electrical signal, a change in electrochemical signal, a change in a color signal, a change in fluorescence signal, a change in a luminescence signal, an acoustic change, and a change in density, and combinations thereof.

The following terms are defined below.

The term "antibody", which is also referred to in the art as "immunoglobulin" (Ig), as used herein refers to a protein constructed from paired heavy and light polypeptide chains; various Ig isotypes exist, including IgA, IgD, IgE, IgG, and IgM. When an antibody is correctly folded, each chain folds into a number of distinct globular domains joined by more linear polypeptide sequences. For example, the immunoglobulin light chain folds into a variable (V_{L}) and a constant (C_{L}) domain, while the heavy chain folds into a variable (V_{H}) and three constant (C_{H}, C_{H2}, C_{H3}) domains. Interaction of the heavy and light chain variable domains (V_{H} and V_{L}) results in the formation of an antigen binding region (Fv). Each domain has a well-established structure familiar to those of skill in the art.

The light and heavy chain variable regions are responsible for binding the target antigen and can therefore show significant sequence diversity between antibodies. The constant regions show less sequence diversity, and are responsible for binding a number of natural proteins to elicit important biochemical events. The variable region of an antibody contains the antigen-binding determinants of the molecule, and thus determines the specificity of an antibody for its target antigen. The majority of sequence variability occurs in six hypervariable regions, three each per variable heavy (V_{H}) and light (V_{L}) chain; the hypervariable regions combine to form the antigen-binding site, and contribute to binding and recognition of an antigenic determinant. The specificity and affinity of an antibody for its antigen is determined by the structure of the hypervariable regions, as well as their size, shape, and chemistry of the surface they present to the antigen. Various schemes exist for identification of the regions of hypervariability, the two most common being those of Kabat and of Chothia and Lesk [Kabat et al, 1991, J Immunol (1991) 147(5):1709-1719; Chothia and Lesk 1987, J Mol Biol (1987) 196(4):901-917], define the "complementarity-determining regions" (CDR) based on sequence variability at the antigen-binding regions of the V_{H} and V_{L} domains. Chothia and Lesk 1987, J Mol Biol (1987) 196(4):901-917 define the "hypervariable loops" (H or L) based on the location of the structural loop regions in the V_{H} and V_{L} domains. These individual schemes define CDR and hypervariable loop regions that are adjacent or overlapping, those of skill in the antibody art often utilize the terms "CDR" and "hypervariable loop" interchangeably, and they may be so used herein. The CDR/loops are identified herein according to the Kabat scheme (i.e. CDR1, 2 and 3, for each variable region).

An "antibody fragment" or an "antigen binding domain", or an "antigen binding fragment" as referred to herein may include any suitable antigen-binding antibody fragment known in the art. The antibody fragment may be a naturally-occurring antibody fragment, or may be obtained by manipulation of a naturally-occurring antibody or by using recombinant methods. For example, an antibody fragment may include, but is not limited to a Fv, single-chain Fv (scFv; a molecule consisting of V_{L} and V_{H} connected with a peptide linker), Fab, F(ab')₂, single-domain antibody (sdAb; a fragment composed of a single V_{L} or V_{H}), and multivalent presentations of any of these. Antibody fragments such as those just described may require linker sequences, disulfide bonds, or other type of covalent bond to link different portions of the fragments; those of skill in the art will be familiar with the requirements of the different types of fragments and various approaches and various approaches for their construction.

In a non-limiting example, the antibody fragment may be an sdAb derived from naturally-occurring sources. Heavy chain antibodies of camelid origin (Hamers-Casterman et al, 1993, Nature 363: 446-448) lack light chains and thus their antigen binding sites consist of one domain, termed V_{H}H. sdAb have also been observed in shark and are termed V_{NAR} (Nuttall et al, 2003, Eur. J. Biochem. 270: 3543-3554). Other sdAb may be engineered based on human Ig heavy and light chain sequences (Jespers et al, 2004, Nat. Biotechnol. 22: 1161-1165; To et al, 2005, J. Biol. Chem. 280: 41395-41403). As used herein, the term "sdAb" includes those sdAb directly isolated from V_{H}, V_{H}H, V_{L}, or V_{NAR} reservoir of any origin through phage display or other technologies, sdAb derived from the aforementioned sdAb, recombinantly produced sdAb, as well as those sdAb generated through further modification of such sdAb by humanization, affinity maturation, stabilization, solubilization, camelization, or other methods of antibody engineering. Also encompassed by the present invention are homologues, derivatives, or fragments that retain the antigen-binding function and specificity of the sdAb.

SdAb possess desirable properties for antibody molecules, such as high thermostability, high detergent resistance, relatively high resistance to proteases (Dumoulin et al, 2002, Protein Sci. 11: 500-15) and high production yield (Arbabi-Ghahroudi et al, 1997); they can also be engineered to have very high affinity by isolation from an immune library (Li et al, 2009, Mol. Immunol. 46: 1718-1726) or by *in vitro* affinity maturation (Davies & Riechmann, 1996, Immunotechnology 2: 169-79). Further modifications to increase stability, such as the introduction of non-canonical disulfide bonds (Hussack et al, 2011a,b; Kim et al, 2012, J. Biol. Chem. 286: 8961-8976), may also be brought to the sdAb.

A person of skill in the art would be well-acquainted with the structure of a single-domain antibody (see, for example, 3DWT, 2P42 in Protein Data Bank). An sdAb comprises a single immunoglobulin domain that retains the immunoglobulin fold; most notably, only three CDR/hypervariable loops form the antigen-binding site. However, and as would be understood by those of skill in the art, not all CDR may be required for binding the antigen. For example, and without wishing to be limiting, one, two, or three of the CDR may contribute to binding and recognition of the antigen by the sdAb of the present invention. The CDR of the sdAb or variable domain are referred to herein as CDR1, CDR2, and CDR3.

The term "nucleic acid" is intended to mean the biopolymers, or small biomolecules, essential to all known forms of life. The term nucleic acid is intended to include DNA and RNA, and may also encompass PNA. They are composed of nucleotides, which are the monomers made of three components: a 5-carbon sugar, a phosphate group and a nitrogenous base. If the sugar is a compound ribose, the polymer is RNA (ribonucleic acid); if the sugar is derived from ribose as deoxyribose, the polymer is DNA (deoxyribonucleic acid). In the case of PNA, the polymer is artificially synthesized and is similar to DNA or RNA.

The term "nucleic acid structure" is intended to mean a structure that is assembled (built, constructed) from the annealed motifs obtained from the annealing of the individual oligonucleotides used in the present invention. The interaction of these oligonucleotides through the full or partial base pairing of nucleotides or peptides provides, ultimately, the nucleic acid structure.

The term "domain" is intended to mean a segment of an oligonucleotide having a defined length and sequence, that is complementary to another domain of another oligonucleotide involved in the assembly of an annealed motif involved in the assembly of a nucleic acid structure of the present invention.

The term "motif' is intended to mean a nucleotide pattern that is widespread and/or repeated through the nucleic acid structure of the present invention.

The term "sensor" is intended to mean a device which detects or measures a physical property and records, indicates, or otherwise responds to it.

The term "nucleic acid junction(s)" or "junction" is intended to mean annealed (double stranded) nucleic acids that intersect with other annealed (double stranded) nucleic acids to form junctions. These nucleic acid structures comprise three-way junctions, four way junctions, five way junctions and six way junctions, as non-limiting examples. These junctions may comprise un-annealed bases between the annealed (double stranded) nucleic acids that intersect. These intersecting annealed (double stranded) nucleic acids share strands.

The term "functional element" is intended to mean generic features of the nucleic acid structure that carry out a specific function or functions, such as recognition of an analyte, interaction with other segments of the nucleic acid structure, etc.

The term "aptamer" is intended to mean oligonucleotide or peptide molecules that bind to a specific target molecule. Aptamers are usually created by selecting them from a large random sequence pool, but natural aptamers also exist in riboswitches. Aptamers can be combined with ribozymes, DNAzymes or peptidases to self-cleave in the presence of their target molecule.

Features and advantages of the subject matter hereof will become more apparent in light of the following detailed description of selected embodiments, as illustrated in the accompanying figures. As will be realized, the subject matter disclosed and claimed is capable of modifications in various respects, all without departing from the scope of the claims. Accordingly, the drawings and the description are to be regarded as illustrative in nature, and not as restrictive and the full scope of the subject matter is set forth in the claims.

### BRIEF DESCRIPTION OF THE DRAWINGS

Further features and advantages of the present disclosure will become apparent from the following detailed description, taken in combination with the appended drawings.
Fig. 1A illustrates a single oligonucleotide that may be used in the nucleic acid structure of the present invention.

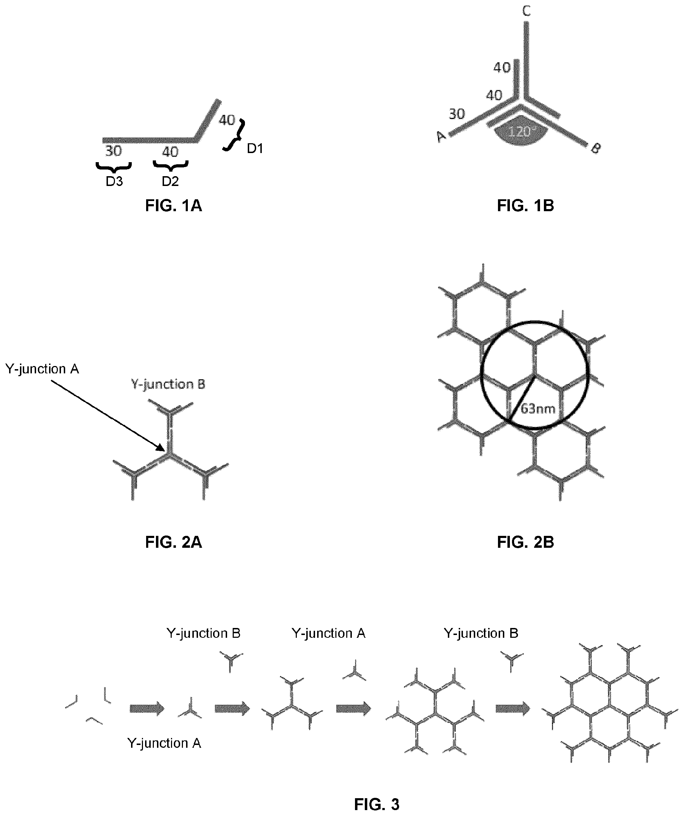

Fig. 1B illustrates the general 3 oligonucleotides structural design allowing the annealed motif formation. In this example, the structure is made of short nucleic acid (e.g. DNA) oligonucleotides featuring (a) at least 3 contiguous or discontinuous sequences referred to as first, second and third domains: the third domain being a 30 bp sticky end region for base pairing to a complementary annealed motif's third domain, and two 40 bp sequences forming the main structure of the annealed motif (the first and second domains) producing binding between oligos A, B, and C as shown in Fig. 1B. The annealed motif structure based on the known properties of DNA measures 10.5 bp per turn, and 0.23-0.33 nm/bp (based on the conformation of A or B DNA). The diameter of the arms of the annealed motif is approximately 2.3 nm. Based on the 110bp oligonucleotide length each oligonucleotide is around 25.3 nM in length. While this structure represents the general form used in the present invention, attachment of the annealed motif to the surface or to receptors/detection molecules requires one of the strands to be broken into shorter sequences of about 40 bp and 70 bp with the 40 bp sequence providing an anchor modification for attachment to the surface or an attachment sequence for receptor binding at the center of the Y junctions.
Fig. 2A illustrates assembled annealed motifs. annealed motifs A, as in Fig. 1 may act as an anchor structure on the sensor surface providing sticky ends for the binding of a second annealed motifs B . This larger structure depicts three annealed motifs B structures hybridized to an annealed motif A.
Fig. 2B illustrates the subsequent alternating additions of Y annealed motifs A and B which produce a larger honeycomb like structure. In embodiments, every annealed motifs B may have a receptor attachment site producing a radius of 63 nm between receptor attachment sites.
Fig. 3 illustrates annealed motifs for the production of uniform hexagonal honeycomb surface tiling. Schematic representation of the sequential additions required to form the nucleic acid structure for sensor surfaces.
Fig. 4A illustrates Atomic Force Microscopy showing assembly of hexagonal honey comb like structures as a regular DNA surface. The image suggests the proper assembly of the structured DNA surface.
Fig. 4B illustrates Atomic Force Microscopy showing assembly of hexagonal honey comb like structures as a regular DNA surface. The image is a zoomed in version to highlight the honeycomb like structure.
Fig. 5 illustrates the sequential annealed motifs additions building mass on the surface of a Surface Plasmon Resonance chip. Using a Surface Plasmon Resonance device to analyze a chip coated with the nucleic acid structure described herein, is showing the sequential addition of hexagonal DNA structure forming the honey comb nucleic acid structure.
Fig. 6 illustrates the alternating pattern of anchor Y junctions (comprising receptors/detection molecules) used for attachment of the structure to the sensor surface and annealed motifs used for receptor attachment. This construction allows the nucleic acid structure to covalently bind on the chip or sensor surface. Solidly anchored, the nucleic acid structure will maintain regularity and homogeneity to the detection device.
Fig 7A shows representative images of the binding/anchoring structures on the annealed motif to bind the nucleic acid structure to the sensor surface.

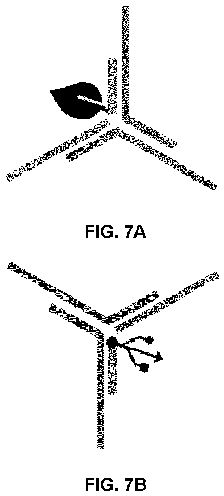

Fig 7B shows representative images of the binding/anchoring structures on the annealed motif to capture receptors/detection molecules such as antibodies, aptamers, oligonucleotides, chemical hooks, dyes, nanoparticles, etc. The receptors/detection molecules may be made of 4 oligonucleotides instead of three in this context, while preserving the basic functionality of the annealed motif. The fourth oligonucleotide may harbor overhanging elements such as, but not limited to nanotubes, quantum dots, metals, silicon, etc., using modifications such as, but not limited to an oligonucleotide, a peptide, a thiol, an amide, a diazonium, an azido, an alkyne, biotin, and receptors or linker element such as but not limited to an oligonucleotide, a peptide, a thiol, an amide, a diazonium, an azido, an alkyne, biotin, etc. to capture other nucleic acids, or any aptamers to take a specific conformation to bind, capture, link to specific molecules, or proteins, etc. to bind more or less complex molecules or microorganisms.
Fig. 8 illustrates using Surface Plasmon Resonance to monitor generating the nucleic acid structure, the antibodies attachment and the detection of a substrate, such as a bacteria. The surface plasmon resonance technology allowed monitoring the functionality and flexibility of the method, herein by capturing antibodies targeting specific substrates.
Fig. 9 illustrates a multilayered nucleic acid structure. It shows a means of amplifying the sensitivity through the generation of a multilayer nucleic acid structure where additional branched structures projecting from the surface could be used to dramatically multiply the number of detection elements.
Fig 10A illustrates the detection of cocaine. A cocaine specific aptamer is integrated into the annealed motif of the self-assembled nucleic acid structure on the surface of a gold electrode demonstrating a change in signal versus non-functionalized surface.
Fig. 10B illustrates an AFM image of the single layer cocaine aptamer integrated scaffold system.
Fig 11A illustrates using a specific aptamer for cocaine and neomycin detection. Using a specific aptamer attached to the annealed motif as shown in Fig. 10B, the overhanging aptamer is used in the binding and detection of free cocaine in a solution in one case, and free neomycin in a solution in the second case and as illustrated in Fig. 11A, shows the SPR device to monitor the binding of the different elements.
Fig 11B illustrates the multilayer approach shown in Fig. 9, in which the addition of multilayers of the cocaine aptamer surface is used to increase the sensitivity and selectivity of neomycin SPR detection by lowering the detection limit and reducing nonspecific interactions.

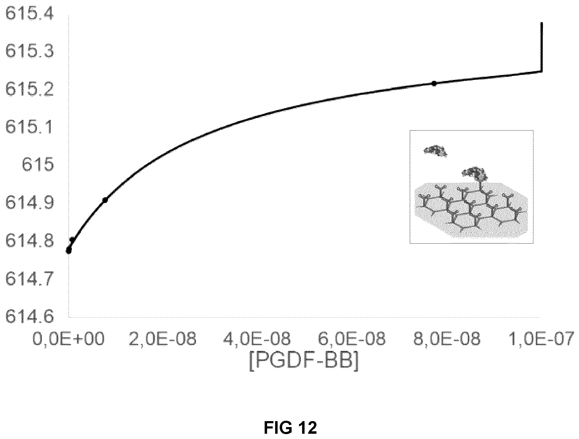

Fig 12 illustrates using the DNA surface to generate a homogenous assembly of PDGF-BB aptamer. Attaching the PDGF-BB aptamer to the honeycomb structure, the binding affinity could be precisely measured. In this case, the lower Kd registered and when compared with the reported value for the free aptamer confirmed that the invention is working properly.

It will be noted that throughout the appended drawings, like features are identified by like reference numerals.

### DETAILED DESCRIPTION

In the current design, a number of different but partly homogenous sequences with a maximum length of 110 base pairs (bp) oligonucleotides are synthesized and purified. In this embodiment, two annealed motifs are formed, one which may be configured for surface attachment and one for receptor or detection molecule attachment. The nucleic acid structure and the annealed motifs self-assembly may be performed by mixing equimolar amounts of the oligonucleotides forming them. Initial covalent attachment may be achieved by seeding the sensor surface with a dilute anchor sequence. This is followed by the sequential addition of oligonucleotides that recognize the anchor sequence and build the seed annealed motifs. Pre-assembled annealed motifs may be subsequently incubated with the surface stepwise to produce a uniform nucleic acid structure tiling.

This unique design allows the positioning of recognition element uniformly and evenly distributed along the surface, with attachment sites that are made available for proper orientation and maximizing of binding receptors such as antibodies.

To provide an overall understanding of the systems, devices, process and methods described herein, certain illustrative implementations will be described. It is to be understood that the systems, process, and methods disclosed herein, while shown for use in a molecule detection systems for the detection of chemical, biological markers, cells and others may be applied to any systems that require analysis.

In this invention, a combination of self-assembled nucleic acid structures of DNA, RNA or PNA layers allows binding sites evenly spaced with regularity and well-structured in a controlled and predictable manner to bind different receptors or molecules developing a biological sensing device. Such construction allows an exact count of the binding detection material, for optimizing and calibrating the biosensor.

According to an embodiment, there is disclosed a nucleic acid structure comprising a plurality of annealed motifs, each of the annealed motifs comprising at least three oligonucleotides, each of the at least three oligonucleotides comprising a respective first, second and third domain, each of the first domain of a respective oligonucleotide of the at least three oligonucleotides being complementary for base pairing with a single one of the second domain of another oligonucleotide of the at least three oligonucleotides, to form the annealed motifs; and each of the third domain of a respective oligonucleotide of the at least three oligonucleotides being complementary for base pairing with the third domain of another oligonucleotide from a different annealed motif, to form nucleic acid structure.

Referring now to the drawings, and more particularly to Fig. 1B, a diagram illustrating a general nucleic acid structure comprised of at least 3 oligonucleotides allowing the formation of a Y junction (annealed motif). In embodiments, the at least three oligonucleotides may have equal lengths or any one of the at least three oligonucleotides may have different lengths. In this embodiment, the structure is comprised of short DNA oligonucleotides featuring (a) 3 contiguous sequences, one 30 base pair (bp) sticky end region for base pairing to a complementary annealed motif (D3) and two 40 bp sequences forming the main structure of the annealed motif (D2 and D1) providing binding between oligonucleotides A, B, and C as shown in Fig. 1B. The first, second, and third domain are oriented in a 5' to 3' orientation: i.e. 5' - D1 - D2 - D3 - 3'. According to an embodiment, the nucleic acid Y junction structure may be based on the known properties of DNA and measures 10.5 base pairs (bp) per turn, and 0.23-0.33 nm/bp (based on the conformation of A or B DNA). In embodiments, any one of the at least three oligonucleotides are from about 18 to about 180 nucleotides in length, or from about 20 to about 180, or from about 25 to about 180, or from about 30 to about 180, or from about 35 to about 180, or from about 40 to about 180, or from about 45 to about 180, or from about 50 to about 180, or from about 55 to about 180, or from about 60 to about 180, or from about 65 to about 180, or from about 70 to about 180, or from about 75 to about 180, or from about 80 to about 180, or from about 85 to about 180, or from about 90 to about 180, or from about 95 to about 180, or from about 100 to about 180, or from about 110 to about 180, or from about 120 to about 180, or from about 130 to about 180, or from about 140 to about 180, or from about 150 to about 180, or from about 160 to about 180, or from about 170 to about 180, or from about 18 to about 170 nucleotides in length, or from about 20 to about 170, or from about 25 to about 170, or from about 30 to about 170, or from about 35 to about 170, or from about 40 to about 170, or from about 45 to about 170, or from about 50 to about 170, or from about 55 to about 170, or from about 60 to about 170, or from about 65 to about 170, or from about 70 to about 170, or from about 75 to about 170, or from about 80 to about 170, or from about 85 to about 170, or from about 90 to about 170, or from about 95 to about 170, or from about 100 to about 170, or from about 110 to about 170, or from about 120 to about 170, or from about 130 to about 170, or from about 140 to about 170, or from about 150 to about 170, or from about 160 to about 170, or from about 18 to about 160 nucleotides in length, or from about 20 to about 160, or from about 25 to about 160, or from about 30 to about 160, or from about 35 to about 160, or from about 40 to about 160, or from about 45 to about 160, or from about 50 to about 160, or from about 55 to about 160, or from about 60 to about 160, or from about 65 to about 160, or from about 70 to about 160, or from about 75 to about 160, or from about 80 to about 160, or from about 85 to about 160, or from about 90 to about 160, or from about 95 to about 160, or from about 100 to about 160, or from about 110 to about 160, or from about 120 to about 160, or from about 130 to about 160, or from about 140 to about 160, or from about 150 to about 160, from about 18 to about 150 nucleotides in length, or from about 20 to about 150, or from about 25 to about 150, or from about 30 to about 150, or from about 35 to about 150, or from about 40 to about 150, or from about 45 to about 150, or from about 50 to about 150, or from about 55 to about 150, or from about 60 to about 150, or from about 65 to about 150, or from about 70 to about 150, or from about 75 to about 150, or from about 80 to about 150, or from about 85 to about 150, or from about 90 to about 150, or from about 95 to about 150, or from about 100 to about 150, or from about 110 to about 150, or from about 120 to about 150, or from about 130 to about 150, or from about 140 to about 150, or from about 18 to about 140 nucleotides in length, or from about 20 to about 140, or from about 25 to about 140, or from about 30 to about 140, or from about 35 to about 140, or from about 40 to about 140, or from about 45 to about 140, or from about 50 to about 140, or from about 55 to about 140, or from about 60 to about 140, or from about 65 to about 140, or from about 70 to about 140, or from about 75 to about 140, or from about 80 to about 140, or from about 85 to about 140, or from about 90 to about 140, or from about 95 to about 140, or from about 100 to about 140, or from about 110 to about 140, or from about 120 to about 140, or from about 130 to about 140, or from about 18 to about 130 nucleotides in length, or from about 20 to about 130, or from about 25 to about 130, or from about 30 to about 130, or from about 35 to about 130, or from about 40 to about 130, or from about 45 to about 130, or from about 50 to about 130, or from about 55 to about 130, or from about 60 to about 130, or from about 65 to about 130, or from about 70 to about 130, or from about 75 to about 130, or from about 80 to about 130, or from about 85 to about 130, or from about 90 to about 130, or from about 95 to about 130, or from about 100 to about 130, or from about 110 to about 130, or from about 120 to about 130, or from about 18 to about 120 nucleotides in length, or from about 20 to about 120, or from about 25 to about 120, or from about 30 to about 120, or from about 35 to about 120, or from about 40 to about 120, or from about 45 to about 120, or from about 50 to about 120, or from about 55 to about 120, or from about 60 to about 120, or from about 65 to about 120, or from about 70 to about 120, or from about 75 to about 120, or from about 80 to about 120, or from about 85 to about 120, or from about 90 to about 120, or from about 95 to about 120, or from about 100 to about 120, or from about 110 to about 120, or from about 18 to about 110 nucleotides in length, or from about 20 to about 110, or from about 25 to about 110, or from about 30 to about 110, or from about 35 to about 110, or from about 40 to about 110, or from about 45 to about 110, or from about 50 to about 110, or from about 55 to about 110, or from about 60 to about 110, or from about 65 to about 110, or from about 70 to about 110, or from about 75 to about 110, or from about 80 to about 110, or from about 85 to about 110, or from about 90 to about 110, or from about 95 to about 110, or from about 100 to about 110, or from about 18 to about 100 nucleotides in length, or from about 20 to about 100, or from about 25 to about 100, or from about 30 to about 100, or from about 35 to about 100, or from about 40 to about 100, or from about 45 to about 100, or from about 50 to about 100, or from about 55 to about 100, or from about 60 to about 100, or from about 65 to about 100, or from about 70 to about 100, or from about 75 to about 100, or from about 80 to about 100, or from about 85 to about 100, or from about 90 to about 100, or from about 95 to about 100, or from about 18 to about 95 nucleotides in length, or from about 20 to about 95, or from about 25 to about 95, or from about 30 to about 95, or from about 35 to about 95, or from about 40 to about 95, or from about 45 to about 95, or from about 50 to about 95, or from about 55 to about 95, or from about 60 to about 95, or from about 65 to about 95, or from about 70 to about 95, or from about 75 to about 95, or from about 80 to about 95, or from about 85 to about 95, or from about 90 to about 95, or from about 18 to about 90 nucleotides in length, or from about 20 to about 90, or from about 25 to about 90, or from about 30 to about 90, or from about 35 to about 90, or from about 40 to about 90, or from about 45 to about 90, or from about 50 to about 90, or from about 55 to about 90, or from about 60 to about 90, or from about 65 to about 90, or from about 70 to about 90, or from about 75 to about 90, or from about 80 to about 90, or from about 85 to about 90, or from about 18 to about 85 nucleotides in length, or from about 20 to about 85, or from about 25 to about 85, or from about 30 to about 85, or from about 35 to about 85, or from about 40 to about 85, or from about 45 to about 85, or from about 50 to about 85, or from about 55 to about 85, or from about 60 to about 85, or from about 65 to about 85, or from about 70 to about 85, or from about 75 to about 85, or from about 80 to about 85, or from about 18 to about 80 nucleotides in length, or from about 20 to about 80, or from about 25 to about 80, or from about 30 to about 80, or from about 35 to about 80, or from about 40 to about 80, or from about 45 to about 80, or from about 50 to about 80, or from about 55 to about 80, or from about 60 to about 80, or from about 65 to about 80, or from about 70 to about 80, or from about 75 to about 80, or from about 18 to about 75 nucleotides in length, or from about 20 to about 75, or from about 25 to about 75, or from about 30 to about 75, or from about 35 to about 75, or from about 40 to about 75, or from about 45 to about 75, or from about 50 to about 75, or from about 55 to about 75, or from about 60 to about 75, or from about 65 to about 75, or from about 70 to about 75, or from about 18 to about 70 nucleotides in length, or from about 20 to about 70, or from about 25 to about 70, or from about 30 to about 70, or from about 35 to about 70, or from about 40 to about 70, or from about 45 to about 70, or from about 50 to about 70, or from about 55 to about 70, or from about 60 to about 70, or from about 65 to about 70, or from about 18 to about 65 nucleotides in length, or from about 20 to about 65, or from about 25 to about 65, or from about 30 to about 65, or from about 35 to about 65, or from about 40 to about 65, or from about 45 to about 65, or from about 50 to about 65, or from about 55 to about 65, or from about 60 to about 65, or from about 18 to about 60 nucleotides in length, or from about 20 to about 60, or from about 25 to about 60, or from about 30 to about 60, or from about 35 to about 60, or from about 40 to about 60, or from about 45 to about 60, or from about 50 to about 60, or from about 55 to about 60, or from about 18 to about 55 nucleotides in length, or from about 20 to about 55, or from about 25 to about 55, or from about 30 to about 55, or from about 35 to about 55, or from about 40 to about 55, or from about 45 to about 55, or from about 50 to about 55, or from about 18 to about 50 nucleotides in length, or from about 20 to about 50, or from about 25 to about 50, or from about 30 to about 50, or from about 35 to about 50, or from about 40 to about 50, or from about 45 to about 50, or from about 18 to about 45 nucleotides in length, or from about 20 to about 45, or from about 25 to about 45, or from about 30 to about 45, or from about 35 to about 45, or from about 40 to about 45, or from about 18 to about 40 nucleotides in length, or from about 20 to about 40, or from about 25 to about 40, or from about 30 to about 40, or from about 35 to about 40, or from about 18 to about 35 nucleotides in length, or from about 20 to about 35, or from about 25 to about 35, or from about 30 to about 35, or from about 18 to about 30 nucleotides in length, or from about 20 to about 30, or from about 25 to about 30, or from about 18 to about 25 nucleotides in length, or from about 20 to about 25, or from about 18 to about 20 nucleotides in length.

The diameter of the arms of the nucleic acid Y junctions is approximately 2.3 nm. Based on the 110 bp oligonucleotide length, each sequence is about 25.3 nm in length. While this structure represents the general form used in the invention, attachment of the nucleic acid structure to surfaces or receptors may be achieved by having one of the strands to be broken into a sequence of 40 bp and 70 bp, with the 40 bp sequence comprising an anchoring modification for attachment to the surface or an attachment sequence for receptor binding, for example at the center of the Y junctions.

According to an embodiment, eight oligonucleotides with a maximum length of 110 bp are synthesized and purified. In embodiments, two annealed motifs according to the present invention are formed: one for surface attachment and the other for receptor attachment. The annealed motif self-assembly was performed by mixing equimolar DNA (each one of the individual oligonucleotides) component strands. Initial covalent attachment is achieved by seeding the sensor surface with a dilute anchor oligonucleotide. This is followed by the sequential addition of oligonucleotides that recognize the anchor oligonucleotides in high salt buffer and building the seed annealed motifs. Pre-annealed motifs are subsequently incubated with the surface stepwise in high salt buffer to produce a resulting nucleic acid structure having a "3-Y" shape joined at a central stem (See Fig. 2A) and which eventually form a uniform hexagonal honeycomb surface tiling (See Fig. 2B). Figs. 1 and 2 show that the selection of 110 bp oligonucleotides is recommended to generate hexagonal honeycomb-like structures of the specific dimensions and of 63 nm diameter. According to an embodiment, it is acceptable and possible to use longer or shorter oligonucleotides as long as the at least three defined domains constituting the design of each oligonucleotides is respected to provide the desired assembly into annealed motifs and ultimately the nucleic acid structures. In embodiments, changing the total length of the oligonucleotides will change and customize the honeycomb structure radius.

Due to this unique design, recognition element-target interactions generate uniform evenly distributed attachment sites that are then made available for proper orientation and maximizing of binding receptors such as antibodies.

Now referring to Fig. 2, there is shown an embodiment of annealed motifs assembly. An annealed motif (See Y junction A in Fig. 2A) is initially formed through seed structures on the sensor surface, which provides attached ends for the binding of an annealed motif referred to as Y junction B, which when exposed to attached Y junction A, produces a structure composed of one Y junction A and three Y junction B (Fig. 2A). This is then exposed to a solution containing preassembled Y junction A which adds 6 Y junction A's to the structure and forms the initial hexagonal conformations of the seeds nucleic acid structures shown in Fig. 2B. As the surface is exposed to alternating solutions of annealed motifs Y junction A and B the honeycomb continues to grow and form links between the initial seed points on the sensor surface. With the sensor surface coverage every 63 nm harbors a binding site for DNA elements, aptamers, proteins, peptides, etc.

According to an embodiment, an important step in the forming of DNA surfaces is to assemble the Y junctions using sequential addition of the strands of annealed motif Y-junction A, which builds initially sparsely seeded Y-junction A and subsequently alternating the addition of pre-assembled Y-junctions B, to eventually produce the honeycomb like pattern; this pattern produces 7 attachment sites in a radius of approximately 63 nm. Now referring to Fig. 3, there is shown a summary of the nucleic acid structure self-assembling sensor surface.

Therefore, according to embodiments, the nucleic acid structure of the present invention may further comprise a first anchoring moiety, for anchoring of the nucleic acid structure to a solid support, such as the sensor surface.

According to another embodiment, the nucleic acid structure may further comprise a second anchoring moiety, for anchoring a functional element to the nucleic acid structure.

In embodiments, the first or second anchoring moiety may be a thiol group, an amide group, a diazonium group, an azido group, an alkyne group, a nanotube, a quantum dot, a metal, a silicon, an oligonucleotide, a peptide, a biotin, and combinations thereof. For example, the oligonucleotide may be a polyT(25), a polyA(25), or any oligonucleotides complementary to another oligonucleotide on the surface of the solid support and/or of the functional element that will be coupled to it. According to some embodiments, the first or second anchoring moiety may be attached to an oligonucleotide having a nucleotide sequence complementary for the first domain or the second domain of another oligonucleotide of the annealed motif. According to an embodiment, the first or the second anchoring moiety may further comprise a functional element.

According to another embodiment, the functional element may comprise a nucleic acid moiety, a protein moiety, a peptide moiety, a polysaccharide moiety, a microorganism moiety, or combinations thereof. For example, the nucleic acid moiety may be an aptamer domain.

According to another embodiment, any one of the first, second and third domain, or a combination thereof, of any one of the at least three oligonucleotides may further comprises an aptamer domain. According to an embodiment, the aptamer domain may be configured to bind to an antigen.

In an embodiment, the nucleic acid moiety may be a nucleic acid structure according to the present invention; the protein moiety may be an antibody, an antigen binding domain thereof, or a fusion protein thereof.

According to an embodiment, an annealed motif from the plurality of annealed motifs may form a functional element, and may therefore perform both a structural role as well as a functional role in the nucleic acid structure of the present invention. In another embodiment, an annealed motif from the plurality of annealed motifs may further comprise a functional element incorporated in any oligonucleotide of the annealed motif. In an embodiment, the functional element is an aptamer domain.

According to another embodiment, the covalent immobilization of antibodies (as functional elements) on various surfaces have been reported ranging from silane linkers on hydroxylated surfaces and thiol monolayers on gold to functional polymers and hydrogels. More recently, various DNA nanostructures have emerged as surface supports. Several strategies have been used to bind proteins at specific locations on DNA nanoscaffolds, such as biotin-streptavidin interaction; antibody-antigen interaction; aptamer binding, Ni(II)-NTA-hexahistidine interaction, and hybridization with DNA-tethered proteins. However, even for antibodies labeled at a specific site, the high flexibility of linkers offers very limited control over the orientation of the antibody. Hence, the development of methods to control the local orientation of antibodies would offer great advances for sensing, structure determination and studies of multivalent interactions.

According to another embodiment there is disclosed that 2 separate annealed motifs (Y-junctions) that bind each other, one annealed motifs that contains a thiol group for Au attachment, and another annealed motifs contains a poly T(25) for aptamer attachment. The specific sequence was generated with a random sequence generator and the sequences were linked together and submitted to the RNA Vienna software to confirm complementarity.

In embodiments, the deposition of the annealed motifs is associated with thiolated DNA anchor for initial annealed motif, then sequential addition of complementary annealed motifs in 1M NaCl buffer is provided as each addition is allowed to hybridize for at least an hour.

According to another embodiment, the solid support on which the nucleic acid structure may be deposited may be a metallic surface, a silicon surface, a polymer surface, and combinations thereof. Examples of metallic surface include a gold surface, a platinum surface, an iron surface, a steel surface, a copper surface, or combinations thereof. Examples of silicon surfaces include a quartz surface, a glass surface, a polymerized siloxane surface, or combinations thereof. Examples of polymer surfaces include a cellulose surface, a starch surface, a nitrocellulose surface, a chitin surface, a plastic surface. The cellulose may be a carboxymethyl cellulose surface.

According to another embodiment, there is disclosed a surface comprising a solid support and a nucleic acid structure according to the present invention attached thereon.

The surface may be a metallic surface, a silicon surface, a polymer surface, and combinations thereof. Examples of metallic surface include a gold surface, a platinum surface, an iron surface, a steel surface, a copper surface, or combinations thereof. Examples of silicon surfaces include a quartz surface, a glass surface, a polymerized siloxane surface, or combinations thereof. Examples of polymer surfaces include a cellulose surface, a starch surface, a nitrocellulose surface, a chitin surface, a plastic surface. The cellulose may be a carboxymethyl cellulose surface.

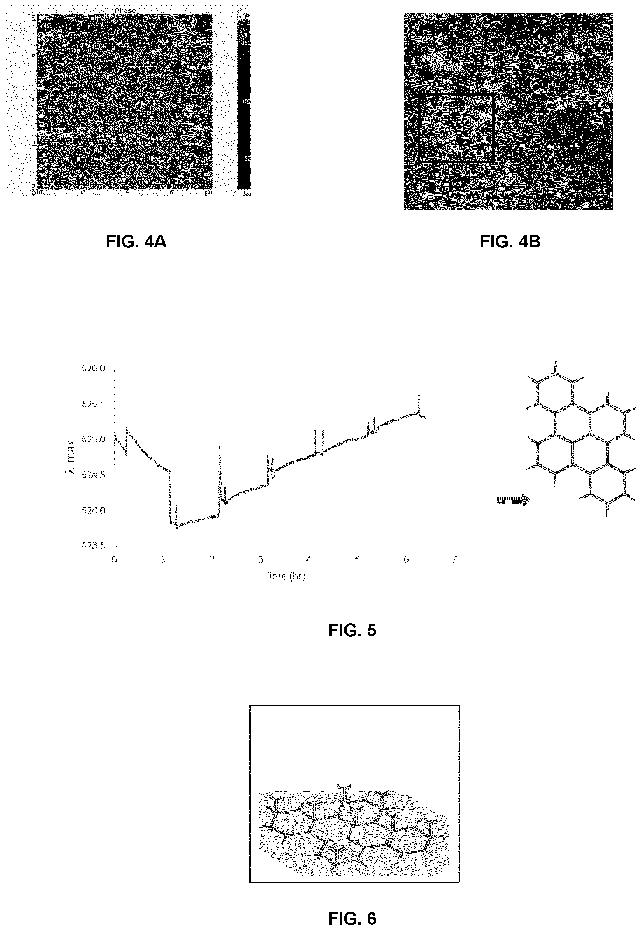

Now referring to Fig. 4, Atomic Force Microscopy is used to visualize the assembled nucleic acid structure Fig. 4A illustrates the general view of the biosensor and Fig. 4B illustrates the honeycomb like surface coverage. This observation confirms the efficacy of the method in generating a single layer of honeycomb-like deoxyribonucleic acid (DNA) structures on the surface of the sensor tool. This method may allow the observation of each antibody attached on the DNA scaffold while it is binding a visible marker.

Now referring to Fig. 5 there is show Surface Plasmon Resonance analysis of DNA tiles addition confirming the structure assembly on the surface. A scaffold is formed from a single layer of a nucleic acid structure of the present invention, which is shown by the shift of SPR resonance over time of addition.

Now referring to Fig. 6, the figure illustrates an alternating pattern of annealed motifs where the junctions of the formed nucleic acid structure are used for attachment of the nucleic acid structure to the sensor surface and other annealed motifs are used for receptor (e.g. antibodies, peptides, or other protein elements) attachment.

Now referring to Fig. 7, the figure illustrates embodiments of the nucleic acid structure to a surface. Fig. 7A shows, using the symbol of a leaf, that chemical groups may be used to bind the nucleic acid structure on the sensor's surface. Fig. 7B shows the branching symbol as representing the different moieties that may be conjugated to the nucleic acid structure of the present invention, including without limitations receptors, normally but without limitation, using a overhanging oligonucleotide fragment that can be, but not limited to, an oligonucleotide poly(T) strand, an aptamer, or others.

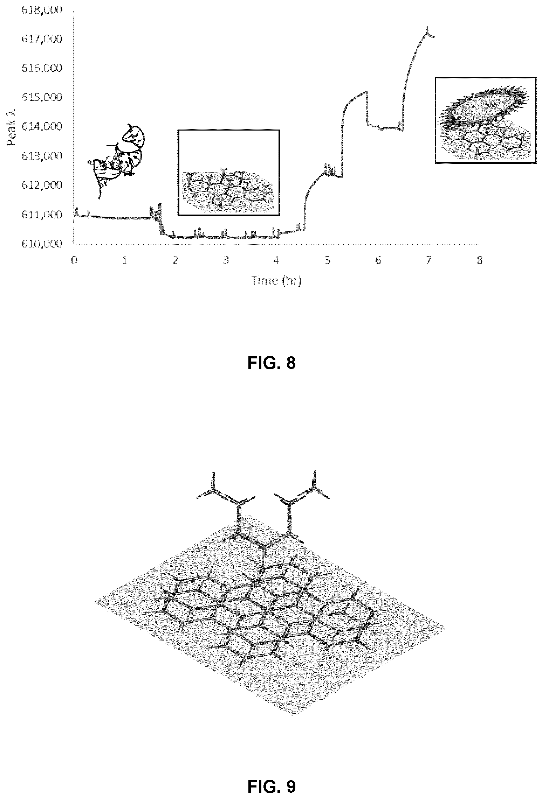

Now referring to Fig. 8, the figure illustrates the use of Surface Plasmon Resonance to monitor generating the nucleic acid structure, the attachment of antibodies as well as the detection of a substrate, such as a bacteria. In this experiment, one may observe that at each step of the nucleic acid structure's assembly, including the addition of the receptor molecules (here antibodies have been used), and the final binding of a substrate bacteria to be detected, and SPR response wavelength shift is observed, clearly demonstrating the efficacy of the method.

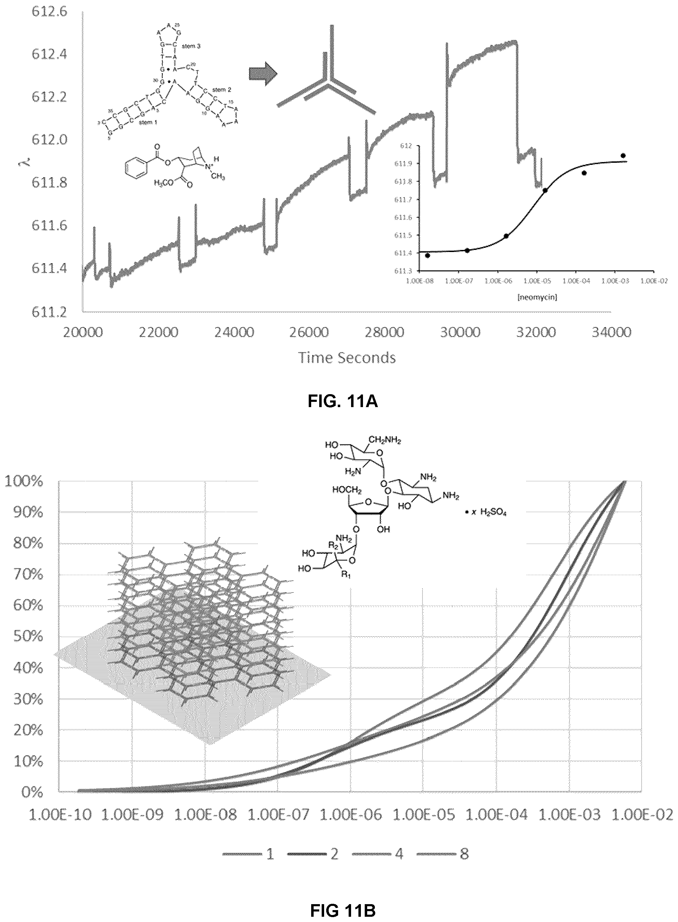

Now referring to Fig. 9, the figure illustrates how the sensitivity of the nucleic acid structure of the present invention may be amplified by the generation of multilayer surfaces and/or multidimensional, as demonstrated in Fig. 11B, from which branched structures based on attached nucleic acid structures of the present invention projecting from the surface to dramatically multiply the number of binding or reacting detection elements.

The use of elements that can link together by hybridization, using specific and unique sequences allows the construction of complex nanostructures with large number of detection sites that can be easily multiplied, thereby greatly increasing the sensitivity of such biosensor.

Therefore, according to another embodiment, there is disclosed a method for producing a nucleic acid structure from at least first and second annealed motifs, each annealed motif comprising at least a first, second and third oligonucleotide each comprising a respective first, second and third domain, each of the first domain of a respective oligonucleotide of the at least a first, second and third oligonucleotide being complementary for base pairing with a single one of the second domain of another of the at least first, second or third oligonucleotide, to form the annealed motif; and at least one of the third domain of a respective oligonucleotide of the at least first, second or third oligonucleotide of the first annealed motif being complementary for base pairing with at least one third domain of one of the at least first, second or third oligonucleotide from the second annealed motif, to form nucleic acid structure, the method comprising step a) :
a) mixing in alternation an amount of the first annealed motif with the second annealed motif for a time sufficient to form the nucleic acid structure.

According to an embodiment, one of the first or the second annealed motif may be an anchored annealed motif, anchored to a solid support.

In an embodiment, the anchored annealed motif is anchored to the solid support by step a') before step a):
a') deposition on the solid support of an oligonucleotide C' having a first domain and a first anchoring moiety, for anchoring of the oligonucleotide C' to a solid support configured to react with the first anchoring moiety,
followed by mixing of an oligonucleotide A having a second domain complementary for base pairing with the first domain of oligonucleotide C',
followed by mixing of an oligonucleotide B having a second domain complementary for base pairing with a first domain of oligonucleotide A, and
followed by mixing of an oligonucleotide C" having a second domain complementary for base pairing with a first domain of oligonucleotide B,
to form the anchored annealed motif;
wherein each of the oligonucleotide A, B and C" have a respective third domain complementary for base pairing with at least one third domain of another oligonucleotide from a different annealed motif.

According to an embodiment, the annealed motif from the at least first and second annealed motifs may form a functional element.

According to another embodiment, the annealed motif from the at least first and second annealed motifs may further comprise a functional element incorporated in any oligonucleotide of the annealed motif.

According to another embodiment, the one of the at least first or the second annealed motif is an anchorage annealed motif further comprises a second anchoring moiety, for anchoring a functional element to the nucleic acid structure.

For example, the functional element may be an aptamer domain. Other examples of functional element comprise a nucleic acid moiety, a protein moiety, a peptide moiety, a polysaccharide moiety, a microorganism moiety, or combinations thereof.

According to another embodiment, there is disclosed a method for preparing an anchorage annealed motif is prepared by step a"
a") mixing an oligonucleotide D' having a first domain and a second anchoring moiety, for anchoring a functional element to the oligonucleotide D',
followed by mixing of an oligonucleotide E having a second domain complementary for base pairing with the first domain of oligonucleotide D',
followed by mixing of an oligonucleotide F having a second domain complementary for base pairing with a first domain of oligonucleotide E, and
followed by mixing of an oligonucleotide D" having a second domain complementary for base pairing with a first domain of oligonucleotide F,
to form the anchorage annealed motif;
wherein each of the oligonucleotide D", E and F have a respective third domain complementary for base pairing with at least one third domain of another oligonucleotide from a different annealed motif.

The first or the oligonucleotide C' or the oligonucleotide D' may further comprises a functional element.

The functional element may comprises a nucleic acid moiety, a protein moiety, a peptide moiety, a polysaccharide moiety, a microorganism moiety, a nanoparticle moiety, or combinations thereof.

According to an embodiment, there is disclosed sensor for the detection of an analyte comprising the nucleic acid motif of the present invention, or the surface of any one of the present invention, in communication with a system for detecting a physical change when the analyte interacts with the nucleic acid motif or the surface. Examples of systems include without limitations surface plasmon resonance (SPR), electrochemical detection using electrodes, colorimetric (such as dip tests like pregnancy tests), spectrometric (requiring a spectrophotometer to detect changes), fluorometric, luminescent, acoustic metric, densitometry, etc. The physical changes that may be detected include without limitations SPR wavelength shift (light interacting with the functionalized metal surface), electrochemical (conductivity, impedance, etc.), colorimetric, fluorometric, and luminescent (changes in light absorption emittance which can be detected visually or with equipment), acoustic metric (change in the penetration/transmission of acoustic waves), densitometry (changes in density at a surface that affect signal transmission such as light), and more.

The physical change may be a change in surface plasmon resonance, a change in electrical signal, a change in fluorescence signal, and a change, and combinations thereof.

According to an embodiment, there is disclosed method of detecting an analyte comprising detecting a physical change with a sensor comprising the nucleic acid motif of the present invention, or the surface of any one of the present invention, in communication with a system for detecting the physical change.

The physical change may be a change in surface plasmon resonance, a change in electrical signal, a change in fluorescence signal, and a change, and combinations thereof.

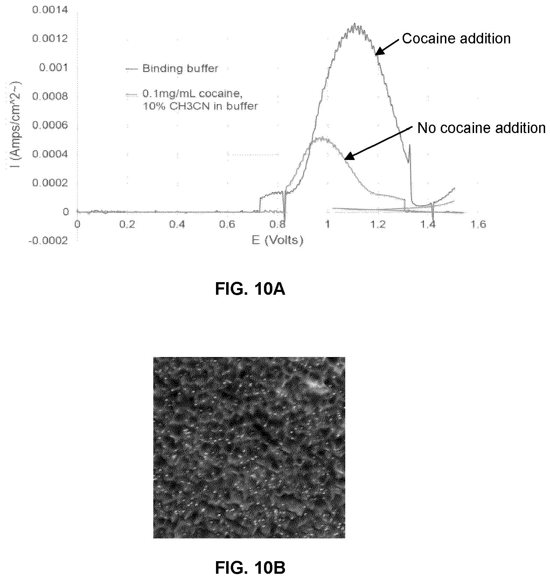

Now referring to Fig. 10A, the figure illustrates the detection of cocaine. A cocaine specific aptamer is incorporated in the one of the annealed motif of the self-assembled nucleic acid structure on the surface of a gold electrode. The passage of a solution comprising 0.1 mg/ml of cocaine shows a change in signal versus non-functionalized surface.

To further demonstrate the usefulness of the present invention, experimental models have been used, using a biosensor surface coated with a multilayer of nucleic acid structures of the present invention on which have been bound aptamers specific to cocaine and neomycin (See Fig. 11), as well as the nucleic acid structure bound with antibodies for the detection of *E*. *coli* bacteria. These experiments measured binding using a surface plasmon resonance apparatus, or using an electrochemical measurement system, and demonstrates a better signal detection associated with higher sensitivity in detecting molecules and microorganisms in the environment.

The present invention will be more readily understood by referring to the following examples which are given to illustrate the invention rather than to limit its scope.

### EXAMPLE 1

### COCAINE DETECTION ON GOLD ELECTROCHEMICAL ELECTRODE

Combining self-assembling nucleic acid structures of the present invention and aptamers for cocaine detection, Fig. 10 illustrates the detection of cocaine using a cocaine specific aptamer incorporated in an annealed motif of the self-assembled nucleic acid structure on the surface of a gold electrode. The signal obtained clearly showed a change in signal versus non-functionalized surface.

The Cocaine aptamer surface was designed by initially producing the sequence for two annealed motifs based on three 55 bp oligonucleotides, where 18 bp domains were used to stabilize the internal structure of the annealed motif (i.e. as first and second domains) and 19 bp domains were used as third domains (i.e. sticky ends) for binding to the other annealed motif. Once the nucleic acid structure was designed, one of the annealed motif was used as the attachment/anchor structure by splitting one of the 55 bp oligonucleotide at the center of the Y junction to create an 18 bp oligonucleotide sequence and a 37 bp oligonucleotide sequence. The 18 bp oligonucleotide sequence was subsequently modified at the 3' end with a thiol group to allow attachment to a gold surface. The center of the other annealed motif was then replaced with the sequence of the cocaine aptamer to integrate the aptamer into the structure of the DNA scaffold.

| **TABLE I** | | |
|---|---|---|
| **Sequences of DNA Aptamers on Scaffold elements** | | |
| Annealed motif A cocaine apt | | |
| Seq ID No: 1 | Seq1a YJA CocaApt | GGCGTGCGCGTTCCATG-SH |
| Seq ID No: 2 | Seq1b YJA CocaApt | AAACCTGTCATAACTTACTGTCCTGATCGGAAGGATC |
| Seq ID No: 3 | Seq2 YJA CocaApt | GTAAGTTATGACAGGTTTCTAGATCTTTGCTCACGCTGTCC TGATCGGAAGGATC |
| Seq ID No: 4 | Seq3 YJA CocaApt | GCGTGAGCAAAGATCTAGACATGGAACGCGCACGCCTGTC CTGATCGGAAGGATC |

| Annealed motif B cocaine apt | | |
|---|---|---|
| Seq ID No: 5 | Seq1 YJB CocaApt | CTGTAGTGAGTTCGAGACAAGGACCATTGCATGCGAGATC CTTCCGATCAGGACA |
| Seq ID No: 6 | Seq2 YJB CocaApt | TCGCATGCAATGGTCCTTCAATGATATCCCTGGATGGATCC TTCCGATCAGGACA |
| Seq ID No: 7 | Seq3 YJB CocaApt | CATCCAGGGATATAGTGGGTCGAGAACTCACTACAGGATC CTTCCGATCAGGACA |

In the example, using the oligonucleotides of Table I, the surface deposition is according the following steps. Mix 2 mL 100 mM aqueous thiolated Y-junction A seq 1a (SEQ ID NO:1), with 4 mL 50 mM TCEP and incubate for 1 hour at room temperature. Dilute 1/500 with H₂O twice and then mix at a ratio of 6 mL to 394 mL 10 mM citrate buffer 0.5 M NaCl (pH 3) to produce 400 mL of deposition solution per cm² (-2 × 10⁸ molecules/cm²). Deposit on gold substrate and incubate for 30 min. Wash with H₂O followed by washing with 10 mM Tris pH 7.4 1 M NaCl 1 mM EDTA. Add an equivalent volume approximately 400 µl/cm² Y-junction A sequence 3 (SEQ ID NO:4) in 50 nM in 10 mM Tris pH 7.4 1 M NaCl 1 mM EDTA, and incubate for 1 hour. Wash with 10 mM Tris pH 7.4 1 M NaCl 1 mM EDTA. Add 400 µl/cm² Y-junction A sequence 2 (SEQ ID NO:3), 50 nM in 10 mM Tris pH 7.4 1 M NaCl 1 mM EDTA, and incubate for 1 hour. Wash with 10 mM Tris pH 7.4 1 M NaCl 1 mM EDTA. Add 400 µl/cm² Y-junction A sequence 1B (SEQ ID NO:2), 50 nM in 10 mM Tris pH 7.4 1 M NaCl 1 mM EDTA, and incubate for 1 hour to complete the deposition of the annealed motif A seeding structures.

Subsequent additions involve the addition of preassembled annealed motifs which were produced by premixing Seq1a YJA CocaApt (SEQ ID NO:1), Seq1b YJA CocaApt (SEQ ID NO:2), Seq2 YJA CocaApt (SEQ ID NO:3), Seq3 YJA CocaApt (SEQ ID NO:4) to produce Y-junction A at a concentration of 50 nM in 10 mM Tris pH 7.4 1 M NaCl 1 mM EDTA and premixing Seq1 YJB CocaApt (SEQ ID NO:5), Seq2 YJB CocaApt (SEQ ID NO:6) and Seq3 YJB CocaApt (SEQ ID NO:7) to produce annealed motif B at a concentration of 50 nM in 10 mM Tris pH 7.4 1 M NaCl 1 mM EDTA.

Continue by washing the surface with 10 mM Tris pH 7.4 1 M NaCl 1 mM EDTA. Followed by the addition of the incubation of 400 µl of annealed motif B, 50 nM in 10 mM Tris pH 7.4 1 M NaCl 1 mM EDTA, for 1 hour. Wash with 10 mM Tris pH 7.4 1 M NaCl 1 mM EDTA. Add 400 µl annealed motif A, 50 nM in 10 mM Tris pH 7.4 1 M NaCl 1 mM EDTA, and incubate for 1 hour. Wash with 10 mM Tris pH 7.4 1 M NaCl 1 mM EDTA. These steps of annealed motif B and annealed motif B addition are repeated in alternation another 3 times, and then motif B again one last time. Wash with 10 mM Tris pH 7.4 1 M NaCl 1 mM EDTA and optionally add 400 µl 50 mM TCEP and incubate for 1 hour.

The sensing surface was washed with 10mM phosphate buffer 100 mM NaCl pH 7.4. Detection of cocaine or neomycin was performed in the same buffer where the surface was exposed to increasing concentrations of the ligands and response was recorded.

### EXAMPLE 2

### PDGF-BB SPR DETECTION

Attaching the PDGF-BB aptamer to the Honeycomb structure resulted in a binding affinity of 28+/-2 nM.

The hexagonal DNA surface was designed by initially producing the sequence for two annealed motifs based on three 110 bp oligonucleotides, where oligonucleotides sequence lengths of 40 bp domains were used to stabilize the internal structure of the annealed motifs (i.e. as the first and second domains) and 30 bp domains were used as third domains (i.e. sticky ends) for binding to the other annealed motif. Once the nucleic acid structure was designed, one of the annealed motifs was used as the attachment or anchor structure by splitting one of the 110 bp oligonucleotide sequences at the center of the annealed motifs to create a 40 bp oligonucleotide sequence and a 70 bp oligonucleotide sequence. The 40 bp oligonucleotide sequence was subsequently modified at the 3' end with a thiol group to allow attachment to a gold surface at the center of the annealed motifs. The other annealed motif was used for receptor attachment where one of the 110bp oligonucleotide sequences was split at the center of the annealed motifs to create a 40 bp oligonucleotide sequence and a 70 bp oligonucleotide sequence. The 40 bp oligonucleotide sequence was subsequently modified at the 3' end with a 25 bp oligonucleotide poly T sequence to provide complement binding for receptor attachment.

| **TABLE II** | | |
|---|---|---|
| **Sequences of DNA Scaffold elements** | | |
| Y-junction A | | |
| Seq ID No: 8 | Seq1a YJA HEX | CTCTCAAAGTATTATGCAGGACGGCGTGCGCGTTCCATG- SH |
| Seq ID No: 9 | Seq1b YJA HEX | AAACCTGTCATAACTTACCTGAGACTAGTTGGAAGTGTGG CATAGCTTTCATGTCCTGATCGGAAGGATC |
| Seq ID No: 10 | Seq2 YJA HEX | CCACACTTCCAACTAGTCTCAGGTAAGTTATGACAGGTTTC TAGATCTTTGCTCACGCATCTAGTCGGTCCACGTTTGGTC ATAGCTTTCATGTCCTGATCGGAAGGATC |
| Seq ID No: 11 | Seq3 YJA HEX | ACCAAACGTGGACCGACTAGATGCGTGAGCAAAGATCTAG ACATGGAACGCGCACGCCGTCCTGCATAATACTTTGAGAG CATAGCTTTCATGTCCTGATCGGAAGGATC |

| Y-junction B HEX | | |
|---|---|---|
| Seq ID No: 12 | Seq1a YJB HEX | GTTGGCGCCCGACCCTCAGACTCTGTAGTGAGTTCTATGT TTTTTTTTTTTTTTTTTTTTTTTTT |
| Seq ID No: 13 | Seq1b YJB HEX | CCGAGCCATTGCATGCGAGATCGGTAGATTGATAGGGGA TGATCCTTCCGATCAGGACATGAAAGCTATG |
| Seq ID No: 14 | Seq2 YJB HEX | ATCCCCTATCAATCTACCGATCTCGCATGCAATGGCTCGG ACAGAATATCCCTGGATGCAATAGACGGACAGCTTGGTAT GATCCTTCCGATCAGGACATGAAAGCTATG |
| Seq ID No: 15 | Seq3 YJB HEX | ATACCAAGCTGTCCGTCTATTGCATCCAGGGATATTCTGT ACATAGAACTCACTACAGAGTCTGAGGGTCGGGCGCCAA CGATCCTTCCGATCAGGACATGAAAGCTATG |

In the example, using the oligonucleotides of Table II, the surface deposition followed, mix 2 µl 100mM aqueous thiolated Y-junction A seq1a HEX (SEQ ID NO:8), with 4 µl 50mM TCEP and incubate for 1 hour at room temperature. Dilute 1/500 with H₂O twice and then mix at a ratio of 6 µl to 394 µl 10 mM citrate buffer 0.5 M NaCl (pH 3) to produce 400 µl of deposition solution per cm² (~2×10⁸ molecules/cm²). Deposit on gold substrate and incubate for 30 min. Wash with H₂O followed by washing with 10 mM Tris pH 7.4 1 M NaCl 1 mM EDTA. Add an equivalent volume approximately 400 µl/cm² Y-junction A sequence 3 HEX (SEQ ID NO:11) in 50 nM in 10 mM Tris pH 7.4 1 M NaCl 1 mM EDTA, and incubate for 1 hour. Wash with 10 mM Tris pH 7.4 1 M NaCl 1 mM EDTA. Add 400 µl/cm² Y-junction A sequence 2 HEX (SEQ ID NO:10), 50 nM in 10 mM Tris pH 7.4 1 M NaCl 1 mM EDTA, and incubate for 1 hour. Wash with 10 mM Tris pH 7.4 1 M NaCl 1 mM EDTA. Add 400 µl/cm² Y-junction A sequence 1B HEX (SEQ ID NO:9), 50 nM in 10 mM Tris pH 7.4 1 M NaCl 1 mM EDTA, and incubate for 1 hour to complete the deposition of the first annealed motif seeding structures.

Subsequent additions involve the addition of preassembled annealed motifs which were produced by premixing Seq1a YJA HEX (SEQ ID NO:8), Seq1b YJA HEX (SEQ ID NO:9), Seq2 YJA HEX (SEQ ID NO:10), Seq3 YJA HEX (SEQ ID NO:11) to produce annealed motif A at a concentration of 50 nM in 10 mM Tris pH 7.4, 1 M NaCl and 1 mM EDTA, and premixing Seq1a YJB HEX (SEQ ID NO:12), Seq1b YJB HEX (SEQ ID NO:13), Seq2 YJB HEX (SEQ ID NO:14) and Seq3 YJB HEX (SEQ ID NO:15) to produce annealed motif B at a concentration of 50 nM in 10 mM Tris pH 7.4, 1 M NaCl and 1 mM EDTA.

Continue by washing the surface with 10 mM Tris pH 7.4 1 M NaCl 1 mM EDTA. Followed by the addition of the incubation of 400 µl of annealed motif B, 50 nM in 10 mM Tris pH 7.4 1 M NaCl 1 mM EDTA, for 1 hour. Wash with 10 mM Tris pH 7.4 1 M NaCl 1 mM EDTA. Add 400 µl annealed motif A, 50 nM in 10 mM Tris pH 7.4 1 M NaCl 1 mM EDTA and incubate for 1 hour. Wash with 10 mM Tris pH 7.4 1 M NaCl 1 mM EDTA. These steps of annealed motif B and annealed motif B addition are repeated in alternation another 3 times, and then motif B again one last time.

The sensing surface was then exposed to the Platelet-derived growth factor aptamer which had been modified, as in Table III (SEQ ID NO:16), to include a 25 bp poly A tail at a concentration of 50 nM in 10 mM Tris pH 7.4 1 M NaCl 1 mM EDTA and incubated for an hour.

| **TABLE III** | |
|---|---|
| **Sequences of DNA Modified Aptamer** | |
| Seq ID No: 16 | CACAGGCTACGGCACGTAGAGCATCACCATGATCCTGTGAAAAAAAAAAAAAAAAA AAAAAAAAA |

The sensing surface was washed with 10.1 mM Na₂HPO₄, 1.8 mM KH₂PO₄, 137 mM NaCl and 2.7 mM KCI, 1 mM MgCl₂, pH 7.4. Detection of PDGF was performed in the same buffer where the surface was exposed to increasing concentrations of the ligands and response was recorded.

### EXAMPLE 3

### Neomycin detection on SPR

Combining self-assembling surfaces and a cocaine aptamer also known to bind neomycin, using the sensing capabilities of the Surface Plasmon Resonance made neomycin detectable (MW 614) with an affinity of Kd 10.5 µM. Sufficiently sensitive results.

These results are illustrated in Figs. 10 and 11. As shown in Fig. 11, the increasing intensity over time is a representation of the different binding levels of neomycin on the DNA scaffold.

Multilayered structures produced in Fig. 11B were produced using a modified version of the single layer cocaine aptamer surface (example 1). In this iteration of the structure the annealed motifs used to anchor the structure was modified to include a third domain (sticky end) for attachment of the second layer. Once the monolayer was produced a second layer was added by introducing a new annealed motif which contained a third domain (sticky end) for attachment to the first layer, three third domain (sticky ends) (as described for the first layer) for binding the annealed domain containing the cocaine aptamer and a new third domain (sticky end) for attaching a third layer. Once this connection nucleic acid structure was added, the layer was completed with the addition of the cocaine aptamer containing annealed motif. The third layer could then be added by introducing a new annaled motif which contained a third domain (sticky end) for attachment to the second layer, three third domains (sticky ends) for binding the annealed motif containing the cocaine aptamer and the same third domain (sticky end) that was used to connect the first layer to the second layer. This repetition of third domains (sticky ends) allowed for subsequent layers to be built by alternation of the connection nucleic acid structures between addition of cocaine aptamer containing annealed motifs.

| **Table 4** | | |
|---|---|---|
| **Sequences of DNA used to construct multilayer structures** | | |
| Replacement sequence (Seq 102) for surface attachment Y junction and odd layers addition | | |
| Seq ID No: 17 | Seq1b YJC CocaApt | CTGAACATCCACACTTTAGTAAACCTGTCATAACTTACT GTCCTGATCGGAAGGATC |

| Replacement sequences (Seq ID 1 & 2) Sequences used in connection Y junction for even lavers | | |
|---|---|---|
| Seq ID No: 18 | Coca 2nd layer Seq1a YJA CocaApt | GGCGTGCGCGTTCCATGTCTGAATCGATGCGCGGCTT C |
| Seq ID No: 19 | Coca 2nd layer Seq1b YJA CocaApt | ACTAAAGTGTGGATGTTCAGAAACCTGTCATAACTTACT GTCCTGATCGGAAGGATC |

| Replacement sequence (Seq ID 1) Sequence used with Seq 17 in connection Y junction for odd layers | | |
|---|---|---|
| Seq ID No: 20 | Coca 3rd layer Seq1a YJA CocaApt | GGCGTGCGCGTTCCATGTGAAGCCGCGCATCGATTCA G |

In this example the construction of the initial monolayer was prepared as described previously but with the initial seed annealed motif structures, and subsequent annealed motif additions containing the replacement sequence (Seq ID NO: 17) in annealed motif A. The addition of the second layer was achieved by adding a new annealed motif constructed from SEQ ID NOs: 3, 4, 18 and 19 (annealed motif C) 400 µL 50 nM in 10 mM Tris pH 7.4, 1 M NaCl, 1 mM EDTA, for 1 hour. This was washed with 10mM Tris pH 7.4, 1 M NaCl, 1 mM EDTA followed by the addition of 400 µL annealed motif B, 50 nM in 10 mM Tris pH 7.4, 1 M NaCl, 1 mM EDTA for 1 hour. With the completion of the second layer a third was constructed through the addition of a new annealed motif constructed from SEQ ID NOs: 3, 4, 17, 20 (annealed motif D) 400 µL 50nM in 10mM Tris pH 7.4, 1 M NaCl, 1mM EDTA, for 1 hour. This was washed with 10 mM Tris pH 7.4, 1 M NaCl, 1 mM EDTA followed by the addition of 400 µL annealed motif B, 50 nM in 10 mM Tris pH 7.4, 1 M NaCl, 1 mM EDTA for 1 hour. With the addition of layer three, additional layers could be constructed by using the previously described steps alternating the addition of annealed motifs C and D. To determine the effect increasing layers has on the sensitivity of the sensor, measurements against neomycin were compared using 1, 2, 4 and 8 layers (Fig. 11B).

### EXAMPLE 4

### DNA mesh as PCR fragment selection tool

As Fig. 2B shows, the distance between the annealed motif distance composing the hexagonal DNA structure, being measurable, adjustable, and known, the addition of single stranded oligonucleotide fragment will allow the capture of PCR fragments of specific length, and discriminating from the shorter fragment(s). Consequently, incomplete PCR fragments can be discriminated from the full length fragment.This technology was tested during quantitative real-time polymerase chain reaction (QRT-PCR), to refine and quantify the product amplification.

qPCR may be used to quantitatively measure the amplification of DNA using fluorescent dyes, this method is particularly novel and efficient to improve the qPCR method, greatly improving its accuracy. In this experiment, a 371 bp PCR fragment may be bound to determine the exact concentration as compared with the qPCR reading and a Urea gel, which will demonstrate the number of sub-fragments and a more accurate qPCR read may be extrapolated. The same experiments may be performed with a 200 bp PCR fragment (which was too short to properly bind), and no variation of qPCR reading should be noticeable, as opposed to the 371 bp fragment where a clear quantification should be possible.

While preferred embodiments have been described above and illustrated in the accompanying drawings, it will be evident to those skilled in the art that modifications may be made without departing from this disclosure. Such modifications are considered as possible variants comprised in the scope of the disclosure .

## Claims

1. A nucleic acid structure comprising
a plurality of annealed motifs, each of said annealed motifs comprising at least three oligonucleotides,
each of said at least three oligonucleotides comprising a respective first, second and third domain,
each of said first domain of a respective oligonucleotide of said at least three oligonucleotides being complementary for base pairing with a single one of said second domain of another oligonucleotide of said at least three oligonucleotides, to form said annealed motifs; and
each of said third domain of a respective oligonucleotide of said at least three oligonucleotides being complementary for base pairing with said third domain of another oligonucleotide from a different annealed motif, to form nucleic acid structure.

2. The nucleic acid structure of claim 1, wherein any one of said at least three oligonucleotides comprises at least three domains.

3. The nucleic acid structure of any one of claims 1 - 2, wherein any one of said at least three oligonucleotides has equal length, and preferably any one of said at least three oligonucleotides are 18-180 nucleotides in length.

4. The nucleic acid structure of any one of claims 1 - 3, wherein any one of said first, second and third domain of any one of said at least three oligonucleotides has a different length, and preferably any one of said at least three oligonucleotides are 18-180 nucleotides in length.

5. The nucleic acid structure of any one of claims 1 - 4, further comprising a first anchoring moiety, for anchoring of said nucleic acid structure to a solid support.

6. The nucleic acid structure of any one of claims 1 - 5, further comprising a second anchoring moiety, for anchoring a functional element to said nucleic acid structure.

7. The nucleic acid structure of any one of claims 5-6, wherein said first or said second anchoring moiety is a thiol group, an amide group, a diazonium group, an azido group, an alkyne group, a nanotube, a nanoparticle, a quantum dot, a metal, a silicon, an oligonucleotide, a peptide, a biotin, and combinations thereof, wherein said oligonucleotide is preferably a polyT(25), a polyA(25), or a combination thereof.

8. The nucleic acid structure of any one of claims 5-7, wherein said first or said second anchoring moiety is attached to an oligonucleotide having a nucleotide sequence complementary for said first domain or said second domain of another oligonucleotide of said annealed motif.

9. The nucleic acid structure of claim 8, wherein said first or said second anchoring moiety further comprises a functional element.

10. The nucleic acid structure of any one of claims 6 - 9, wherein said functional element comprises a nucleic acid moiety, a protein moiety, a peptide moiety, a polysaccharide moiety, a microorganism moiety, a nanoparticle moiety, or combinations thereof, and wherein preferably said protein moiety is an antibody, an antigen binding domain thereof, or a fusion protein thereof.

11. The nucleic acid structure of claim 10, wherein said nucleic acid moiety is an aptamer domain, and/or at least one of said nucleic acid structure; and preferably said aptamer domain is configured to bind to an antigen.

12. The nucleic acid structure of any one of claims 1 - 11, wherein any one of said first, second and third domain, or a combination thereof, of any one of said at least three oligonucleotides further comprises an aptamer domain.

13. The nucleic acid structure of any one of claims 1 to 2, wherein an annealed motif from said plurality of annealed motifs forms a functional element.

14. The nucleic acid structure of any one of claims 1 to 12, wherein an annealed motif from said plurality of annealed motifs further comprises a functional element incorporated in any oligonucleotide of said annealed motif.

15. The nucleic acid structure of any one of claims 5 to 14, wherein said solid support is a metallic surface, a silicon surface, a polymer surface, and combinations thereof.

## Patentansprüche

1. Nukleinsäurestruktur, die Folgendes umfasst:
eine Vielzahl von annealten Motiven, wobei jedes der annealten Motive mindestens drei Oligonukleotide umfasst,
jedes der mindestens drei Oligonukleotide eine jeweilige erste, zweite und dritte Domäne umfasst,
jede der ersten Domäne eines jeweiligen Oligonukleotids der mindestens drei Oligonukleotide zur Basenpaarung mit einer einzigen der zweiten Domäne eines anderen Oligonukleotids der mindestens drei Oligonukleotide komplementär ist, um die annealten Motive zu bilden; und
jede der dritten Domäne eines jeweiligen Oligonukleotids der mindestens drei Oligonukleotide zur Basenpaarung mit der dritten Domäne eines anderen Oligonukleotids von einem anderen annealten Motiv komplementär ist, um eine Nukleinsäurestruktur zu bilden.

2. Nukleinsäurestruktur nach Anspruch 1, wobei jedwedes der mindestens drei Oligonukleotide mindestens drei Domänen umfasst.

3. Nukleinsäurestruktur nach einem der Ansprüche 1-2, wobei jedwedes der mindestens drei Oligonukleotide eine gleiche Länge aufweist und bevorzugt jedwedes der mindestens drei Oligonukleotide eine Länge von 18-180 Nukleotiden aufweist.

4. Nukleinsäurestruktur nach einem der Ansprüche 1-3, wobei jedwede der ersten, zweiten und dritten Domäne von jedwedem der mindestens drei Oligonukleotide eine unterschiedliche Länge aufweist und bevorzugt jedwedes der mindestens drei Oligonukleotide eine Länge von 18-180 Nukleotiden aufweist.

5. Nukleinsäurestruktur nach einem der Ansprüche 1-4, die weiter eine erste Verankerungseinheit zur Verankerung der Nukleinsäurestruktur an einem festen Träger umfasst.

6. Nukleinsäurestruktur nach einem der Ansprüche 1-5, die weiter eine zweite Verankerungseinheit zur Verankerung eines funktionellen Elements an der Nukleinsäurestruktur umfasst.

7. Nukleinsäurestruktur nach einem der Ansprüche 5-6, wobei die erste oder die zweite Verankerungseinheit Folgendes ist: eine Thiolgruppe, eine Amidgruppe, eine Diazoniumgruppe, eine Azidogruppe, eine Alkingruppe, ein Nanoröhrchen, ein Nanopartikel, ein Quantenpunkt, ein Metall, ein Silicium, ein Oligonukleotid, ein Peptid, ein Biotin und Kombinationen davon, wobei das Oligonukleotid bevorzugt ein polyT(25), ein polyA(25) oder eine Kombination davon ist.

8. Nukleinsäurestruktur nach einem der Ansprüche 5-7, wobei die erste oder die zweite Verankerungseinheit an einem Oligonukleotid angebracht ist, das eine Nukleotidsequenz aufweist, die zu der ersten Domäne oder der zweiten Domäne eines anderen Oligonukleotids des annealten Motivs komplementär ist.

9. Nukleinsäurestruktur nach Anspruch 8, wobei die erste oder die zweite Verankerungseinheit weiter ein funktionelles Element umfasst.

10. Nukleinsäurestruktur nach einem der Ansprüche 6-9, wobei das funktionelle Element Folgendes umfasst: eine Nukleinsäureeinheit, eine Proteineinheit, eine Peptideinheit, eine Polysaccharideinheit, eine Mikroorganismeneinheit, eine Nanopartikeleinheit oder Kombinationen davon, und wobei bevorzugt die Proteineinheit ein Antikörper, eine antigenbindende Domäne davon oder ein Fusionsprotein davon ist.

11. Nukleinsäurestruktur nach Anspruch 10, wobei die Nukleinsäureeinheit eine Aptamerdomäne und/oder mindestens eine der Nukleinsäurestruktur ist und bevorzugt die Aptamerdomäne zur Bindung zu einem Antigen konfiguriert ist.

12. Nukleinsäurestruktur nach einem der Ansprüche 1-11, wobei jedwede der ersten, zweiten und dritten Domäne oder eine Kombination davon eines jedweden der mindestens drei Oligonukleotide weiter eine Aptamerdomäne umfasst.

13. Nukleinsäurestruktur nach einem der Ansprüche 1 bis 2, wobei ein annealtes Motiv aus der Vielzahl von annealten Motiven ein funktionelles Element bildet.

14. Nukleinsäurestruktur nach einem der Ansprüche 1 bis 12, wobei ein annealtes Motiv aus der Vielzahl von annealten Motiven weiter ein funktionelles Element umfasst, das in jedwedes Oligonukleotid des annealten Motivs eingefügt ist.

15. Nukleinsäurestruktur nach einem der Ansprüche 5 bis 14, wobei der feste Träger eine metallische Oberfläche, eine Siliciumoberfläche, eine Polymeroberfläche und Kombinationen davon ist.

## Revendications

1. Structure d'acide nucléique comprenant
une pluralité de motifs hybridés, chacun desdits motifs hybridés comprenant au moins trois oligonucléotides,
chacun desdits au moins trois oligonucléotides comprenant un premier, un deuxième et un troisième domaines respectifs,
chacun desdits premiers domaines d'un oligonucléotide respectif desdits au moins trois oligonucléotides étant complémentaire pour l'appariement des bases avec un seul dudit deuxième domaine d'un autre oligonucléotide desdits au moins trois oligonucléotides, pour former lesdits motifs hybridés; et
chacun desdits troisièmes domaines d'un oligonucléotide respectif desdits au moins trois oligonucléotides étant complémentaire pour l'appariement des bases avec ledit troisième domaine d'un autre oligonucléotide provenant d'un motif hybridé différent, pour former la structure d'acide nucléique.

2. La structure d'acide nucléique selon la revendication 1, dans laquelle l'un quelconque desdits au moins trois oligonucléotides comprend au moins trois domaines.

3. La structure d'acide nucléique selon l'une quelconque des revendications 1 - 2, dans laquelle l'un quelconque desdits au moins trois oligonucléotides a une longueur égale, et de préférence l'un quelconque desdits au moins trois oligonucléotides a une longueur de 18 à 180 nucléotides.

4. La structure d'acide nucléique selon l'une quelconque des revendications 1 - 3, dans laquelle l'un quelconque desdits premier, deuxième et troisième domaines de l'un quelconque desdits au moins trois oligonucléotides a une longueur différente, et de préférence l'un quelconque desdits au moins trois oligonucléotides a une longueur de 18 à 180 nucléotides.

5. La structure d'acide nucléique selon l'une quelconque des revendications 1 - 4, comprenant en outre un premier fragment d'ancrage, pour l'ancrage de ladite structure d'acide nucléique à un support solide.

6. La structure d'acide nucléique selon l'une quelconque des revendications 1 - 5, comprenant en outre un second fragment d'ancrage, pour ancrer un élément fonctionnel à ladite structure d'acide nucléique.

7. La structure d'acide nucléique selon l'une quelconque des revendications 5 - 6, dans laquelle ledit premier ou ledit second fragment d'ancrage est un groupe thiol, un groupe amide, un groupe diazonium, un groupe azido, un groupe alcyne, un nanotube, une nanoparticule, un point quantique, un métal, un silicium, un oligonucléotide, un peptide, une biotine et des combinaisons de ceux-ci, ledit oligonucléotide étant de préférence un polyT(25), un polyA(25) ou une combinaison de ceux-ci.

8. La structure d'acide nucléique selon l'une quelconque des revendications 5 - 7, dans laquelle ledit premier ou ledit second fragment d'ancrage est attaché à un oligonucléotide ayant une séquence nucléotidique complémentaire dudit premier domaine ou dudit second domaine d'un autre oligonucléotide dudit motif hybridés.

9. La structure d'acide nucléique selon la revendication 8, dans laquelle ledit premier ou ledit second fragment d'ancrage comprend en outre un élément fonctionnel.

10. La structure d'acide nucléique selon l'une quelconque des revendications 6 - 9, dans laquelle ledit élément fonctionnel comprend un fragment acide nucléique, un fragment protéique, un fragment peptidique, un fragment polysaccharide, un fragment micro-organisme, un fragment nanoparticule, ou des combinaisons de ceux-ci, et dans lequel, de préférence, ledit fragment protéique est un anticorps, un domaine de liaison à l'antigène de celui-ci, ou une protéine de fusion de celui-ci.

11. La structure d'acide nucléique selon la revendication 10, dans laquelle ledit fragment d'acide nucléique est un domaine aptamère, et/ou au moins une desdites structures d'acide nucléique ; et de préférence, ledit domaine aptamère est configuré pour se lier à un antigène.

12. La structure d'acide nucléique selon l'une quelconque des revendications 1-11, dans laquelle l'un quelconque desdits premier, deuxième et troisième domaines, ou une combinaison de ceux-ci, de l'un quelconque desdits au moins trois oligonucléotides comprend en outre un domaine aptamère.

13. La structure d'acide nucléique selon l'une quelconque des revendications 1 - 2, dans laquelle un motif hybridé parmi ladite pluralité de motifs hybridés forme un élément fonctionnel.

14. La structure d'acide nucléique selon l'une quelconque des revendications 1 - 12, dans laquelle un motif hybridé provenant de ladite pluralité de motifs hybridés comprend en outre un élément fonctionnel incorporé dans n'importe quel oligonucléotide dudit motif hybridé.

15. La structure d'acide nucléique selon l'une quelconque des revendications 5 - 14, dans laquelle ledit support solide est une surface métallique, une surface de silicium, une surface polymère et des combinaisons de celles-ci.
